(19) Europäisches Patentamt
European Patent Office
Office européen des brevets

(11) Publication number: 0 058 483 B1

(12) EUROPEAN PATENT SPECIFICATION

(45) Date of publication of patent specification: 15.10.86

(51) Int. Cl.4: A 61 C 5/00, A 61 K 6/08, C 07 F 9/14, C 07 F 9/146, C 07 F 9/42

(21) Application number: 82300432.0

(22) Date of filing: 27.01.82

# (54) Dentin and enamel adhesives.

(30) Priority: 13.02.81 US 234560

(43) Date of publication of application: 25.08.82 Bulletin 82/34

(45) Publication of the grant of the patent: 15.10.86 Bulletin 86/42

(84) Designated Contracting States: CH DE FR GB IT LI NL SE

(58) References cited:
BE-A- 753 580
CH-A- 560 226
CH-A- 572 941
DE-A-1 903 168
DE-A-1 935 630
DE-A-2 207 183
FR-A-2 344 281
US-A-2 871 263

Patent abstracts of Japan, vol. 4, no. 89, 25 June 1980, page 51 C 16
JOURNAL OF GENERAL CHEMISTRY USSR, vol. 48, no. 9, part 1, September 1979 T. V. KIM et al: "Vinyl esters of phosphorus acids: XVI. Chemical properties of l-alkenyl alkyl phosphorchloridites", pages 1791 - 1796

(73) Proprietor: MINNESOTA MINING AND MANUFACTURING COMPANY
3M Center, P.O. Box 33427
St. Paul, Minnesota 55133-3427 (US)

(72) Inventor: Bunker, James E.
2501 Hudson Road P.O. Box 33427
St. Paul Minnesota 55133 (US)

(74) Representative: Baillie, Iain Cameron et al
c/o Ladas & Parry Isartorplatz 5
D-8000 München 2 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 058 483 B1

**Description**

Technical Field

This invention relates to the field of polymerizable compounds. In addition, this invention relates to compositions for use as liners, restoratives, and composites for the repair of teeth, compositions for use in fastening orthodontic brackets or crowns to teeth, and to a method for preparing such compositions.

Background Art

Practitioners in the field of dentistry have long sought polymerizable compositions which would adhere well to dentin. One of the first attempts at bonding to dentin was recorded by Buonocore et al, utilizing a polymerizable mixture containing glycerophosphate dimethacrylate (I):

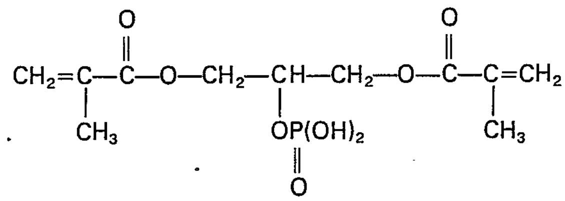

I

see M. Buonocore, W. Wileman, and F. Brudevold, *J. Dent. Res., 35,* 846 (1956), and M. Buonocore and M. Quigley, *J. Amer. Dent. Assoc., 57,* 807 (1958).

Anbar et al have reported dentin adhesives containing vinyl phosphonic acid (II) or vinylbenzyl phosphonic acid (III):

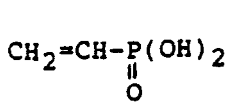

and

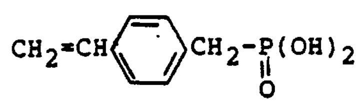

,

II III

see M. Anbar and E. Farley, *J. Dent. Res., 53,* 879 (1974) and E. Farley, R. Jones, and M. Anbar, *J. Dent. Res., 56,* 943 (1977).

Various phosphoric acid and phosphonic acid esters have been described as having good adhesion to dentin in patent applications and patents, see, e.g., U.S. Patent Nos. 4,182,035, 4,222,780, and 4,235,633, O.L.S. No. 2711234, French Patent Application no. 2,344,281, UK published Patent Specification no. 1 569 021 and Japanese laid-open application Nos. 77—113089, 78—30193, 78—39331, 78—67740, 78—69494, 78—110637, 78—113843, 78—134037, 78—144939, 78—138441, 79—21438, and 79—28339. Also there has been introduced in Japan a dental liner composition, under the name "Clearfil", utilizing a two-part resin system. The first (catalyst) portion of such resin system contains a polymerizable phosphoric acid of undetermined structure. The second (universal) part of such resin system contains an ethanolic solution of sodium benzenesulfinate and N,N-dihydroxyethyl-p-toluidine (the latter compound will be referred to hereafter as "DHPT"). It has been recommended that the use of this composition be preceded by acid etching of the exposed dentin (e.g., with ortho-phosphoric acid) prior to application of the liner composition. However, the long term physiological affects of such acid etching are unknown, and the efficacy of acid etching of dentin has been questioned by practitioners, see, e.g., M. G. Buonocore, "The Challenge of Bonding to Dentin", *The Acid Etch Technique,* L. M. Silverstone and I. L. Dogon, Eds., Proceedings of an International Symposium at St. Moritz, Switzerland, Dec. 16—18, 1974, North Central Publishing Co. (St. Paul, 1975). Also, acid etching is a somewhat difficult procedure to carry out, since the highly corrosive acid is injurious to the soft tissues of the mouth. In addition, commercial products containing ortho-phosphoric acid are, in some jurisdictions, subjected to special transportation requirements which increase the costs of shipping dental supplies (e.g. restoration kits) which contain vials of ortho-phosphoric acid.

A non-phosphorus acid compound which is said to possess bonding capability to dentin is reported in U. S. Patent No. 4,203,220. The preferred compound in said patent is 2-N'-allylamino-4,6-dichloro-1,3,5-triazine (IV):

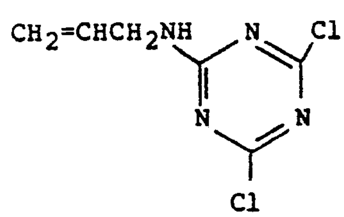

IV

Organic esters of monofluorophosphoric acid having the formula (V):

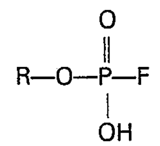

V

wherein R is an unsaturated addition polymerizable group have been described as having good adhesion to hard tooth tissues in U.S. Patent Nos. 3,882,600 and 3,997,504, although no indication regarding the adhesion to dentin of such esters of monofluorophosphoric acid is given in said patents.

In U.S. Patent No. 3,629,187 there are described various adducts for use as dental resins, made by combining diglycidyl methacrylate of Bisphenol A (hereafter referred to as "BIS—GMA") and isocyanate or diisocyanate. Phosphorus-containing adducts are not described in said patents.

U.S. Patent No. 2,674,590 describes various poly-condensation products in which phosphorus atoms are linked to two chain-forming aromatic esterifying groups and to one branched aromatic esterifying group containing a diphenyl group. U.S. Patent No. 2,871,263 describes various phosphoric dihalides in which phosphorus is bonded to two halogen atoms, is doubly bonded to an oxygen atom, and is singly bonded to a monovalent aromatic hydrocarbon radical containing at least one olefinic double bond and having at least one halogen atom attached to a carbon atom therein. U.S. Patent No. 4,030,933 describes phosphorus and halogen containing polymers prepared by reacting a halogenated derivative of bis(hydroxyethyl) terephthalate with a halogen-containing phosphorus monomer, the resulting polymer having repeating units in which halogen is not bonded directly to phosphorus.

A dentin adhesive composition should desirably offer good adhesion to both dentin and tooth enamel, as well as adhering well to other restorative and composite resins, crowns, and/or orthodontic brackets currently in use ("restorative" and "composite" will be used essentially interchangeably herein, in recognition of the fact that due to differing standards currently in effect throughout the world, an individual dental adhesive composition might be regarded as a "restorative" in some jurisdictions and as a "composite" in others). Also, a dentin adhesive composition should desirably reduce the need for detailed cavity preparation such as undercutting. In addition, a dentin adhesive composition should withstand repeated thermally-induced expansion and contraction while minimizing marginal leakage between the adhesive composition and adjacent tooth tissue or restorative or composite materials. Also, it would be desirable if a dentin adhesive composition offered sufficiently strong bonding to dentin and enamel that the acid etching technique currently used for most dental restorations could be eliminated.

Disclosure of Invention

The present invention provides, in one aspect, polymerisable compositions having particularly valuable use in dentistry. Thus there is provided a composition for use in dentistry, characterised by comprising:

(a) at least one polymerisable compound comprising the reaction product of a phosphorus acid having chlorine or bromine bonded directly to phosphorus with a polymerisable monomer having at least one reactive hydroxyl group, and

(b) a sulfur compound in the $^{+2}$ or $^{+4}$ oxidation state.

In another aspect of the invention there is provided a polymerisable composition, characterised by comprising:

(a) at least one polymerizable compound comprising an organic ester of one or more acids of phosphorus, said ester having chlorine or bromine bonded directly to phosphorus, and the organic radical of said ester containing at least one free-radically polymerizable functional group, and

(b) 0.5 to 10 percent by weight sulfur compound in the $^{+2}$ or $^{+4}$ oxidation state.

Optional components in such compositions include a tertiary amine, a free radical initiator, and/or a photoinitiator.

In preferred embodiments the compounds of the invention comprise polymerisable compounds of the formula

$$(R^1{-}O)_m{-}P{-}(Br)_n$$

or

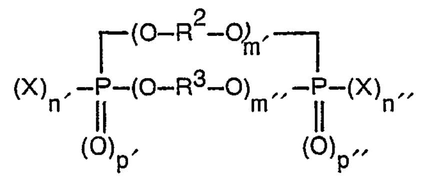

wherein

m and n are at least one,

m+n=3,

m' and m'' are zero or 1 and are the same or different with the proviso that m' and m'' are not both zero,

n′ and n″ are zero to 4 and are the same or different with the proviso that n′ and n″ are not both zero,
p′ and p″ are zero or 1 and are the same or different,
$m'+m''+n'+2p'=3$ or 5,
$m'+m''+n''+2p''=3$ or 5,
$R^1$ is a monovalent, olefinic organic radical
$R^2$ and $R^3$ are divalent, olefinic organic radicals and are the same or different, and
X is Cl, Br, or $R^4$, where $R^4$ is an aliphatic or oxyaliphatic radical having 1 to 12 carbon atoms, and each X is the same as or different from other X, with the proviso that at least one X is Cl or Br.

In one embodiment either or both of $R^1$ and $R^2$ contain skeletal hetero atoms selected from oxygen, sulfur and non-basic nitrogen atoms.

The present invention also provides a method for making compounds of the invention. In particular there is provided a method for making a polymerisable compound for use in dentistry, characterised by mixing BIS—GMA with an acid of phosphorus having chlorine or bromine bonded directly to phosphorus.

In a particular embodiment of the invention there is provided a polymerisable compound useful in dentistry, characterised in that said compound is an organic ester of one or more acids of phosphorus, said ester having chlorine or bromine bonded directly to phosphorus, and the organic radical of said ester containing at least one free-radically polymerisable functional group, wherein said organic radical is the residue remaining after removal of one or more hydroxyl hydrogen atoms from BIS-GMA.

In another aspect of the invention there is provided a two-part polymerisable composition for use as a liner composition, characterised in that the first part comprises the reaction product of BIS—GMA in a diluent with 0.25 to 20 percent by weight phosphorus oxychloride, based on the weight of said BIS—GMA, and a free radical initiator, and the second part comprises a liquid solution comprising sodium benzenesulfinate.

## Detailed Description

In the practice of the present invention, the preferred phosphorus acid esters are prepared by combining a chlorine-containing or bromine-containing phosphorus acid with BIS—GMA. Additional preferred phosphorus acid esters used in the invention can be characterised by the formulas (VI) and (VII):

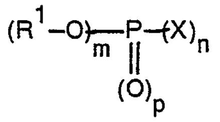

VI

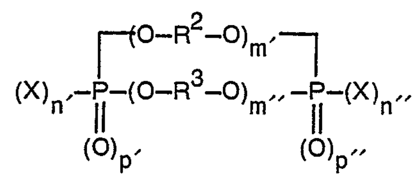

VII

wherein
m is 1 to 3,
m′ and m″ are zero or 1 and are the same or different
n is 1 to 4,
n′ and n″ are independently zero to 4 and are the same or different, with the proviso that n′ and n″ are not both zero,
p, p′ and p″ are zero or 1 and are the same or different,
$m+n+2p=3$ or 5,
$m'+m''+n'+2p'=3$ or 5,
$m'+m''+n''+2p''=3$ or 5
$R^1$ is a monovalent, olefinic organic radical which can be straight chain, or branched, or cyclic, (preferably alkenyl, cycloalkenyl, aralkenyl, or alkenaryl, having 2 to 40 carbon atoms) and can contain skeletal hetero atoms selected from oxygen, sulfur, or non-basic nitrogen atoms.
$R^2$ and $R^3$ are divalent olefinic organic radicals which can be straight chain, branched, or cyclic, (preferably alkenylidene, oxyalkenylidene, cycloalkenylidene, arylenealkenylidene, or alkenylidene-arylene, having 2 to 40 carbon atoms), and can contain skeletal hetero atoms selected from oxygen, sulfur and non-basic nitrogen atoms and are the same or different, and
X is Cl, Br, or $R^4$, where $R^4$ is an aliphatic or oxyaliphatic radical having 1 to 12 carbon atoms, and each X is the same as or different from other X, with the proviso that at least one X is Cl or Br.

Compounds of the formula VI and VII contain trivalent or pentavalent phosphorus atoms. In compounds of formula VI, phosphorus is bonded to at least one chlorine or bromine atom. In compounds of formula VII, at least one phosphorus atom is bonded to at least one chlorine or bromine atom. Preferably phosphorus is bonded to chlorine. Preferably the phosphorus acid esters contain at least one double bond between phosphorus and oxygen. Most preferably two or more polymerisable functional groups per phosphorus atom are contained in the phosphorus acid esters. Also, the phosphorus acid esters are preferably liquids at room temperature.

The polymerisable functional group in the phosphorus acid esters is preferably a free-radically polymerisable group, such as an olefin, and is most preferably a monofunctional or difunctional acryl or methacryl radical. Other polymerisable functional groups include monofunctional or difunctional vinyl, allyl, crotyl, and cinnamyl radicals.

Representative compounds useful in the present invention include:

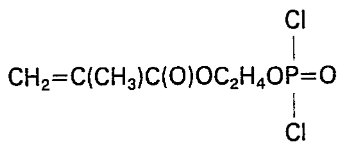 ,

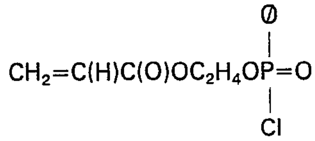 (where "Ø" is a phenyl radical),

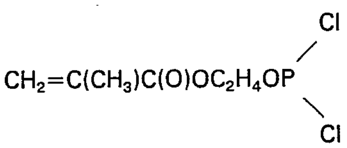 ,

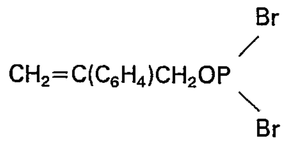 ,

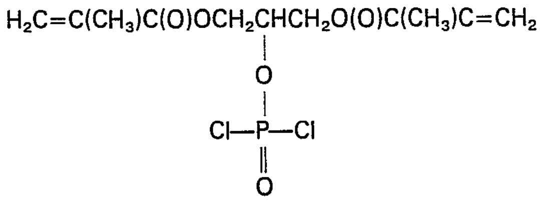 ,

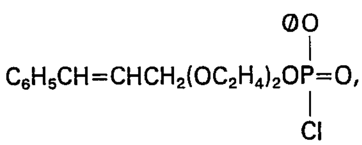

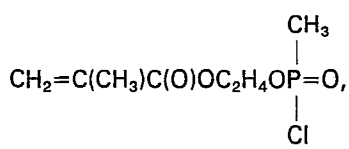

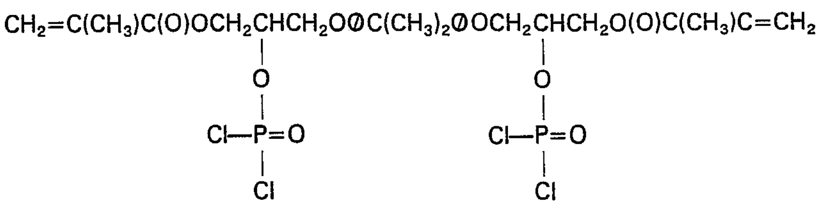 ,

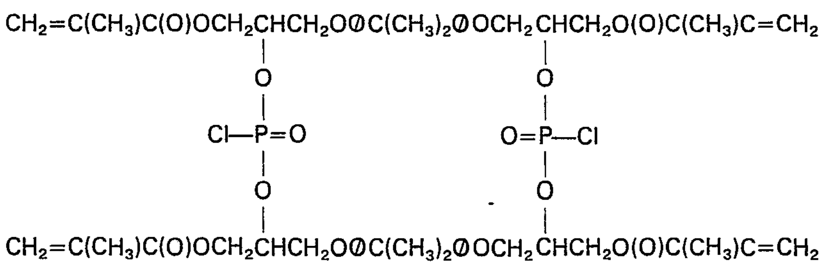 ,

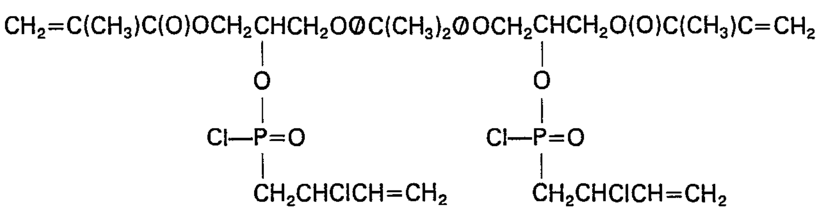

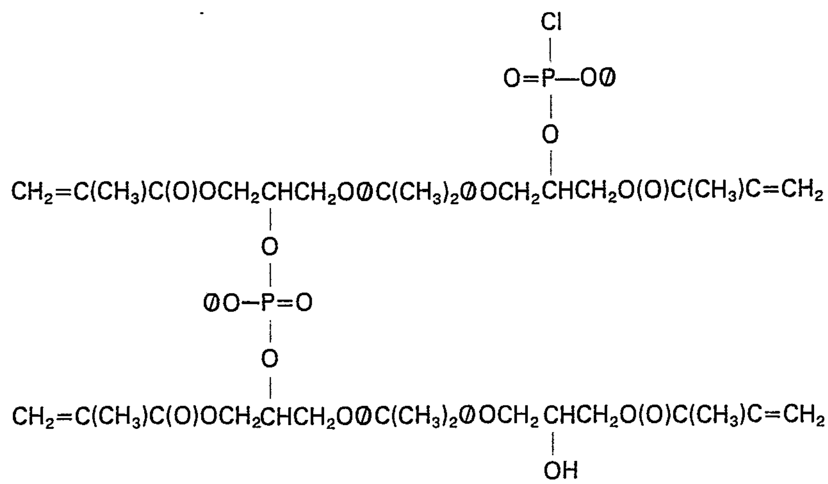
,

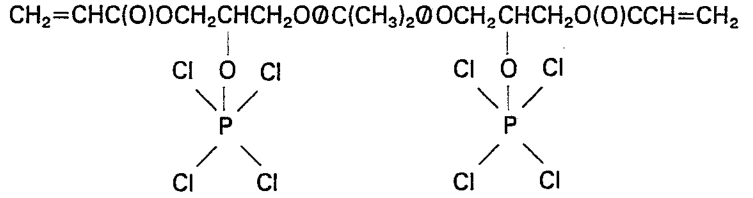
,

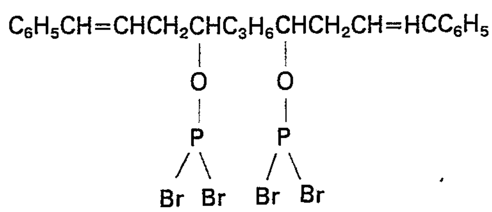
,

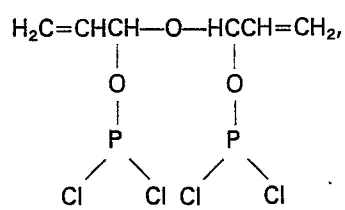
and
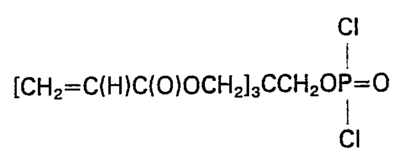
,

as well as mixtures of more than one of the above compounds.

The phosphorus acid esters of the invention can be used individually or in the form of adducts containing more than one phosphorus acid ester of the invention. Preferably, the phosphorus acid esters of the invention are prepared by combining a chlorine-containing or bromine-containing phosphorus acid (e.g., phosphorus oxychloride, $POCl_3$, also known as phosphoryl chloride) with a polymerizable monomer having at least one reactive hydroxyl group (e.g., BIS—GMA). When the polymerizable monomer has a high initial viscosity, it is preferable to mix the phosphorus acid with the polymerizable monomer and a suitable diluent, e.g., triethyleneglycol dimethacrylate.

The phosphorus acid and polymerizable monomer having at least one hydroxyl group will react at low temperature, e.g., at room temperature, and the reaction mixture will increase in viscosity, preferably reaching an equilibrium state that is stable over time. The reaction product of such a mixture will generally be an adduct, the phosphorus acid esters of which are the product of reactions between some or all of the various hydroxyl groups of the polymerizable monomer and available chlorine or bromine atoms of the phosphorus acid. Sufficient phosphorus acid should be added to the polymerizable monomer to provide good bonding and handling performance in liner, restorative, or composite compositions prepared therewith. For an adduct prepared by combining phosphorus oxychloride and BIS—GMA, 0.25 to twenty percent by weight phosphorus oxychloride, and preferably about one to ten percent by weight phosphorus

oxychloride should be used, based on the weight of BIS—GMA. Because BIS—GMA contains two hydroxyl groups per molecule, the above weight percentage values represent equivalent ratios of $POCl_3$ to BIS—GMA of 0.025:1 to 1:1, preferably 0.05:1 to 0.5:1. Suitable adjustment of such equivalent ratios should be made when phosphorus acid esters of this invention are prepared from polymerizable monomers having other hydroxyl functionality, e.g., monofunctionality or trifunctionality. Also, suitable adjustment of such equivalent ratios should be made when phosphorus acid esters of this invention are prepared from phosphorus acids other than phosphorus oxychloride. Expressed in terms of the ratio of halogen atoms in the phosphorus acid to hydroxyl groups in the polymerizable monomer, the phosphorus acid and polymerizable monomer should be combined in a ratio of halogen atom to hydroxyl group between 0.0375:1 to 1.5:1, preferably 0.075:1 to 0.75:1.

If lesser amounts of phosphorus acid than those amounts sufficient to provide good bonding and handling performance are used, the resulting adduct may have low adhesion to dentin and enamel when polymerized therewith. If larger amounts of phosphorus acid than those sufficient to provide good bonding and handling are used, the resulting adduct will tend to homopolymerize, thereby having inadequate shelf life.

Other phosphorus acids which can be reacted with polymerizable monomers having reactive hydroxyl groups include $CH_3POCl_2$, $PCl_3$, $PCl_5$, $C_6H_5POCl_2$, $C_6H_5OPOCl_2$, and $PBr_3$. Such phosphorus acids can be used singly or in combination. Phosphorus oxychloride is a preferred phosphorus acid for use in the preparation of phosphorus acid esters of this invention.

Other polymerizable monomers having reactive hydroxyl groups which can be used in this invention include hydroxyethyl methacrylate, pentaerythritol triacrylate, glycerol dimethacrylate, methylvinyl alcohol, vinylbenzyl alcohol, allyl alcohol, crotyl alcohol, and cinnamyl alcohol.

The mixing of phosphorus acid and polymerizable monomers having reactive hydroxyl groups can be carried out at room temperature. The attainment of equilibrium between the phosphorus acid and polymerisable monomer can be determined by observing the viscosity of the adduct over time, with equilibrium being indicated by a levelling off of such viscosity.

The adhesive strength, resistance to micro-leakage and other characteristics of the phosphorus acid esters of this invention can be evaluated by forming a polymerisable composition containing such phosphorus acid esters together with a sulfur compound having sulfur in the $^{+2}$ or $^{+4}$ oxidation state (with "oxidation state" being defined according to Hendrickson et al, *Organic Chemistry,* 3d Ed., pp 796—799 (McGraw Hill Co., 1970)), a tertiary amine, and a free-radical initiator. The resulting composition is then tested for adhesive strength and resistance to micro-leakage, using the methods outlined in the examples below, and evaluated for toxicity (e.g., cytotoxicity to L—929 mouse fibroblasts) and for other desired characteristics (e.g. shelf life).

The compositions of the invention, particularly for use as liners, oridinarily contain sulfur in the $^{+2}$ or $^{+4}$ oxidation state which acts as activator, in amounts of about 0.5 to 10 percent by weight. Suitable activators are ordinarily alkali metal salts, such as potassium or sodium salts, or ammonium salts, of sulfur-containing anions such as sulfinate, sulfite, or sulfonate anions. Sodium benzenesulfinate is a preferred activator. Such activators can also, if desired, be employed in restorative, composite, and adhesive compositions.

The compositions of the invention, particularly liner, restorative, composite, and adhesive compositions typically contain a tertiary amine which acts as a polymerisation accelerator, in amounts of 0.5 to 10 percent by weight. Suitable tertiary amines include DHPT, N,N-dimethyl-para-toluidine and N,N-bis(2-hydroxyethyl)-3,5-xylidene. DHPT is a preferred tertiary amine. To a certain extent it is believed that the use of accelerator can be avoided in compositions of this invention if sufficient activator (i.e., a sulfur compound having sulfur in the $^{+2}$ or $^{+4}$ oxidation state) is substituted for such accelerator. Accordingly, the present invention includes compositions containing phosphorus acid ester, activator, and catalyst, as well as compositions containing accelerator in addition to such ingredients.

Compositions of the invention for use as liner, restorative, composite and adhesive compositions typically contain a polymerisation catalyst in amounts of 0.05 to 5 percent by weight. Suitable polymerisation catalysts include free-radical initiators such as peroxides, e.g. benzoyl peroxide, acetyl peroxide, lauroyl peroxide, and t-butyl hydroperoxide. Benzoyl peroxide is a preferred catalyst. Photoinitiators (i.e., light-activatable catalysts) such as monoketals of aromatic 1,2-diketones or a combination of benzil and a dialkylamino acrylate or methacrylate can also be used.

When the compositions of the invention are used as liners which are then covered with polymerisable restorative or composite compositions, the liner composition need not contain accelerator or catalyst so long as sufficient accelerator or catalyst can migrate from the polymerisable restorative or composite composition into the liner composition, thereby promoting polymerisation of the resin in the liner. However, for optimum reproducibility in use, liner compositions of this invention typically contain measured amounts of activator, accelerator and catalyst.

Other adjuvants such as solvents, stabilisers, fillers, pigments and inhibitors can also be used in the compositions of this invention. The amounts and types of such adjuvants, and their manner of addition to the compositions of this invention will be essentially the same as currently used in existing liner, restorative, composite, or adhesive compositions familiar to those skilled in the art. Ethanol is a preferred solvent for use in liner compositions of this invention. Quartz, and glasses such as zinc glass or other radiopaque glass treated with appropriate silane surface treatment, are preferred fillers for use in

restorative and composite compositions of this invention. Asbestos-free talc is a preferred filler for use in adhesive compositions of this invention.

The compositions of the invention can be put up in one-part or multiple-part packages. For example, compositions of the invention for use as liners can be one-part packages prepared by combining one or more of the above-described phosphorus acid esters with activator, accelerator, inhibitor, and light-activatable catalyst. The resulting mixture will remain in a stable, essentially uncured state until exposed to suitable radiation, e.g., actinic light radiation. Also, liner compositions can be prepared in one-part packages containing phosphorus acid ester and catalyst but omitting activator or accelerator, and relying on an adjacent layer of polymerisable restorative or composite applied to the teeth to supply such omitted ingredients. The resulting composition will remain in a stable, uncured state until combined with the missing ingredient, e.g., activator migrating into the liner composition from an adjacent layer of polymerisable restorative or composite composition.

Multiple-part packages of liner compositions can be prepared, for example, by combining a suitable solvent (e.g., ethanol), activator, and accelerator in a first part, and phosphorus acid ester and catalyst in a second part. While uncombined, the resulting two-part package will remain in a stable, uncured state. When the two parts are mixed together, e.g., by spatulation or other means, the resulting liner composition will rapidly cure. The amount of each ingredient in such two-part package should be adjusted to allow sufficient working time for the practitioner to mix and apply the liner composition as desired, together with attainment of the desired physical properties in the cured liner.

If desired, other combinations of phosphorus acid ester, activator, accelerator, catalyst, and any other desired adjuvants can also be employed in multiple-part packages of liner compositions of this invention. Preferably, a multiple-part liner composition package offers ease of mixing, good shelf life, and desirable physical properties after cure.

One-part packages for use as restorative, composite, and adhesive compositions can be prepared, for example, by combining one or more of the above-described phosphorus acid esters with accelerator, inhibitor, light-activatable catalyst, and filler. The resulting mixture will remain in a stable, essentially uncured state until exposed to suitable radiation, e.g., actinic light radiation.

Multiple-part packages for use as restorative, composite, and adhesive compositions can be prepared, for example, by combining a polymerizable resin (e.g., BIS—GMA), accelerator, and filler in a first part, and phosphorus acid ester, catalyst, and filler in a second part. While uncombined, the resulting two-part package will remain in a stable, uncured state. When the two parts are mixed together, e.g., by spatulation or other means, the resulting restorative, composite, or adhesive composition will rapidly cure. The amount of each ingredient in such two-part package should be adjusted to allow sufficient working time and attainment of desired physical properties.

If desired, other combinations of polymerizable resin, phosphorus acid ester, activator, accelerator, catalyst, filler, and any other desired adjuvants can also be employed in multiple-part packages of restorative, composite, and adhesive compositions of this invention, coincident with attainment of ease of mixing, good shelf life, and desirable physical properties after cure.

When used as liners, the compositions of this invention are applied in a manner similar to that used for existing dental liner compositions. However, cavity preparation is simplified. Excavation can be limited to the removal of damaged or defective tooth structure. Undercutting of the cavity is generally not required. If desired, acid etching of the cavity can be omitted. This invention therefore shortens the time required for completion of a dental restoration and reduces trauma to healthy tooth structure.

When used as a composite or restorative, the compositions of this invention are used in a fashion similar to that used for existing dental composites and restoratives. Preferably, where the compositions of this invention are used as composites or restoratives, they are used in conjunction with a liner prepared according to this invention which is applied to the excavated cavity prior to application of the composite or restorative composition.

When used as an orthodontic bracket adhesive, the compositions of this invention are preferably used as primers in conjunction with existing filled orthodontic bracket adhesives. The compositions of the invention can also be combined with fillers and used in place of such adhesives, preferably in conjunction with a liner prepared according to the present invention. Where desired, e.g., to obtain very high bonding strength, acid etching of the exposed tooth enamel can be employed. However, satisfactory results can often be obtained in the absence of such acid etching, thereby reducing damage to enamel.

The following examples are offered to aid understanding of the present invention and are not to be construed as limiting the scope thereof.

Example 1

Preparation of organic ester of phosphorous oxychloride

10 g of phosphorus oxychloride was dissolved in a polymerizable monomer mixture containing 95 g of BIS—GMA, 2.0 g of benzoyl peroxide, 95 g of triethyleneglycol dimethacrylate, 0.13 g of butylated hydroxytoluene, 0.34 g of phenyl salicylate, and 0.24 g of glycidyl methacrylate. The resulting mixture was allowed to stand at room temperature for 5 days. During this time, the reaction mixture underwent a gradual increase in viscosity from about 200 cps to 1,345 cps, measured at 24°C. No further change in viscosity was apparent upon further standing. Infrared analysis of the resulting product established that the

number of hydroxyl groups had diminished. The presence of carbonyl, phenyl, and phosphate groups was also established by IR spectrum analysis. Nuclear magnetic resonance spectroscopy established that the product contained a mixture of phosphate esters.

The above reaction product was used as the first part of a two-part liner composition. The second part of such liner composition was a solution of three percent by weight sodium benzenesulfinate and one percent by weight DHPT in ethanol. Adhesion of this liner composition to unetched dentin was evaluated using the following procedure. Four bovine teeth of similar age and appearance were partially embedded in circular acrylic disks. The exposed portion of each tooth was ground flat and parallel to the acrylic disk using 120 grit silicon carbide paper-backed abrasive mounted on a lapidary wheel, in order to expose the tooth dentin. During this and subsequent grinding and polishing steps, the teeth were continuously rinsed with water. Further grinding and polishing of the teeth was carried out by mounting 400 grit silicon carbide paper-backed abrasive, and 600 grit alumina rubber-backed abrasive on the lapidary wheel.

The teeth were then washed with distilled water using a "Water Pik" apparatus set on "hard" for 15 seconds, followed by drying with air. One drop of each part of the above two-part liner composition was placed on a mixing pad. The drops were mixed together by hand spatulation for about 5 seconds, painted onto the polished tooth surface, and blown into a thin film with compressed air. A previously prepared Teflon® mold having a 5 mm diameter hole lined with a gelatin sleeve was clamped around the tooth so that the central axis of the hole in the mold was normal to the polished, liner-coated tooth surface. The cavity in the mold was filled with a standard, premixed dental composite ("Concise" brand, commercially available from 3M). The tooth and mold were allowed to stand for about 10 minutes at room temperature. The mold was then carefully removed from the tooth, leaving a button-like molded composite shape attached to the liner layer. The disk-tooth-liner-composite combination was stored in distilled water at 37°C for 24 hours.

Adhesion of the liner composition to the polished, unetched bovine dentin was evaluated by placing the tooth mounting disk in a holder and clamping the holder in the jaws of an "Instron" apparatus with the liner layer parallel to the direction of pull. A loop of orthodontic wire (0.44 mm diameter) was placed around the composite button adjacent to the polished tooth surface. The ends of the orthodontic wire were clamped in the pulling jaws of the Instron apparatus, thereby placing the liner bond in shear stress. At a crosshead speed of 5 mm/min, the average measured shear strength of the liner-dentin bond was 51 kg/cm$^2$. If allowed to stand in 37°C distilled water for 42 hours (instead of 24 hours), the average measured strength of the liner-dentin bond was 60 kg/cm$^2$.

Using the above technique, the liner bond strength on polished bovine enamel was also evaluated, both with and without acid etching for 1 minute with 37% ortho-phosphoric acid. Bond strength on acid etched enamel was an average 370 kg/cm$^2$, and bond strength on unetched enamel was an average of 120 kg/cm$^2$.

The above-described liner composition was also evaluated for resistance to thermal cycling. Six samples of the liner composition on unetched dentin, and six samples on unetched enamel, prepared as described above, were thermally cycled between 12° and 46°C for 500 cycles. Adhesion values were then measured as described above. After cycling, the average liner bond strength on unetched dentin was 40 kg/cm$^2$, and the average liner bond strength on unetched enamel was 88 kg/cm$^2$.

In a comparison run, "Clearfil" liner (commercially available from the Kuraray Co., Ltd.), was similarly evaluated. The initial average bond strength of "Clearfil" liner was 26 kg/cm$^2$ on unetched dentin, 200 kg/cm$^2$ on etched enamel, and 50 kg/cm$^2$ on unetched enamel. After thermal cycling as described above, the average bond strength of "Clearfil" liner was 13 kg/cm$^2$ on unetched dentin and 21 kg/cm$^2$ on unetched enamel.

## Example 2

Shelf stability

The composition of the invention shown in Example 1 was stored at 45°C for 4 weeks. When the stored composition was mixed and tested as in Example 1, the initial average liner bond strength values were within 0.3 kg/cm$^2$ of the average values of Examples 1.

## Examples 3—26

Adhesion value of other compositions of invention to unetched dentin

Using the method of Example 1, different types and amounts of phosphorus acids were combined with the polymerizable monomer mixture. Set out below in Table 1 are the example number, phosphorus acid used to form the organic ester, the weight percent of such acid added to the polymerizable monomer mixture of Example 1, and the bond strengths of the resulting liner compositions when applied to unetched dentin.

TABLE I

| Example Number | Phosphorus acid | Weight Percent* | Bond strength Kg/cm² |
|---|---|---|---|
| 3 | $POCl_3$ | 0.1 | 3 |
| 4 | $POCl_3$ | 0.25 | 30 |
| 5 | $POCl_3$ | 0.5 | 54 |
| 6 | $POCl_3$ | 1.0 | 43 |
| 7 | $POCl_3$ | 1.5 | 48 |
| 8 | $POCl_3$ | 2.0 | 58 |
| 9 | $POCl_3$ | 2.5 | 53 |
| 10 | $POCl_3$ | 3.0 | 45 |
| 11 | $POCl_3$ | 3.5 | 48 |
| 12 | $POCl_3$ | 4.0 | 51 |
| 13 | $POCl_3$ | 4.5 | 45 |
| 14 | $POCl_3$ | 6.0 | 43 |
| 15 | $POCl_3$ | 6.5 | 35 |
| 16 | $POCl_3$ | 7.5 | 27 |
| 17 | $POCl_3$ | 8.0 | 25 |
| 18 | $POCl_3$ | 8.5 | 31 |
| 19 | $POCl_3$ | 9.1 | 25 |
| 20 | $POCl_3$ | 10 | 26 |
| 21 | $C_6H_5POCl_2$ | 5 | 10 |
| 22 | $C_6H_5OPOCl_2$ | 5 | 40 |
| 23 | $PCl_3$ | 2.5 | 30 |
| 24 | $PCl_3$ | 5 | 39 |
| 25 | $PCl_5$ | 5 | 16 |
| 26 | $PBr_3$ | 2.5 | 42 |

Example 27

Using the method of Example 1, a two-part liner composition was prepared. The first part of the liner composition was prepared by mixing 0.5 g $POCl_3$, 0.12 g benzoyl peroxide, and 9.5 g pentaerythritol triacrylate. The second part of the liner composition was a solution of three percent by weight sodium benzene sulfinate and one percent by weight DHPT in ethanol. The composition was evaluated as in Example 1, and had an average shear strength on unetched dentin of 17 kg/cm².

Example 28

Using the method of Example 27, a two-part liner composition was prepared, using a mixture of 0.5 g of $POCl_3$, 0.057 g benzoyl peroxide, 0.0095 g Bisphenol A, 0.0029 g butylated hydroxytoluene, 4.68 g

triethyleneglycol dimethacrylate, and 4.75 g pentaerythritol triacrylate. The average shear strength of the polymerized resin was 12 kg/cm$^2$.

Example 29

A two-part liner composition containing a monofunctional methacrylate was prepared. The first part of the liner composition was prepared by adding 38.3 g (0.25 moles) $POCl_3$ to a stirred, ice-cooled solution of 25.3 g triethylamine (0.25 moles) in 80 ml tetrahydrofuran and 60 ml ether. To the resulting mixture was added dropwise 32.5 g (0.25 moles) 2-hydroxyethyl methacrylate. The reaction mixture was stirred for about 2 hours, filtered, and the filter cake washed with ether. The resulting red colored filtrate was concentrated to yield an oil weighing 60.5 g and having the formula:

$$\begin{array}{r} Cl \\ | \\ CH_2{=}C(CH_3)C(O)OC_2H_4OP{=}O \\ | \\ Cl \end{array}$$

A 5 g portion of this oil was added to a mixture of 98.54 g triethyleneglycol dimethacrylate, 0.062 g butylated hydroxytoluene, 0.2 g Bisphenol A, and 1.2 g benzoyl peroxide.

The second part of the liner composition was a solution of three percent by weight sodium benzenesulfinate and one percent by weight DHPT in ethanol. The composition was evaluated as in Example 1, and had an average shear strength on unetched dentin of 5 kg/cm$^2$.

Example 30

Example 29 was repeated using 0.15 moles $POCl_3$, 0.30 moles triethylamine, and 0.30 moles 2-hydroxyethyl methacrylate, thereby producing the compound:

$$\begin{array}{c} O \\ \| \\ CH_2{=}C(CH_3)C(O)OC_2H_4OPOC_2H_4O(O)C(CH_3)C{=}CH_2 \\ | \\ Cl \end{array}$$

A liner composition prepared and evaluated as in Example 29 had an average shear strength on unetched dentin of 9 kg/cm$^2$.

Example 31

The liner compositions of the invention shown in Examples 1, 9, 16, and 19 were used as primers for orthodontic bracket adhesives. The resins were mixed and applied to tooth enamel which had been previously polished with 600 grit silicon carbide paper-backed abrasive, washed with a "Water Pik", and air-dried. A layer of standard orthodontic bracket adhesive ("Concise 1960" brand, commercially available from 3M) was applied to the back of an orthodontic bracket and pad (Bracket No. 007 and Pad No. 065, commercially available from American Orthodontics, Inc.). The adhesive-coated pad was applied to the primer and the resulting assembly allowed to cure for ten minutes at room temperature. The cured assemblies were stored in water at 37°C for 24 hours and then evaluated for average shear strength using the "Instron" apparatus described in Example 1. The results are shown below.

| Primer, from resin of Example | Average Shear Strength Kg/cm$^2$ |
|---|---|
| 1 | 88 |
| 9 | 85 |
| 16 | 91 |
| 19 | 78 |

In a comparison run, standard "Enamel Bond" primer from the "Concise 1960" kit was used in place of the above primers. The average shear strength was 31 kg/cm$^2$. Acid etching for one minute with 37% orthophosphoric acid increased the average shear strength using "Enamel Bond" primer to 125 kg/cm$^2$.

This example shows that the compositions of this invention can be used as primers in conjunction with a standard orthodontic bracket adhesive, to provide very high average shear strengths without the use of acid etching.

Comparative Example 1

An organic ester of monofluorophosphoric acid described in U.S. Patent No. 3,997,504 was prepared as described in said patent, and then evaluated for adhesion to dentin as in Example 1.

Pure difluorophosphoric acid was obtained following the procedure of P. A. Bernstein et al, *Inorg. Chem. 10,* 1549 (1971), by cooling 87.4 g of impure difluorophosphoric acid to 0°C. The cooled acid was slowly added to a flask made of "Monel" metal, containing 38 g of $P_2O_5$. The resulting mixture was allowed to stand with occasional shaking for 1 hour at 0°C. The mixture was then slowly pumped through a trap cooled to −78°C with acetone and "Dry Ice". After about 1.5 hours, 13 g of pure difluorophosphoric acid was collected. The pure acid was a clear, fuming liquid.

A 4.8 g portion of pure difluorophosphoric acid was mixed dropwise with 4.0 g 2-hydroxyethyl methacrylate, and allowed to react overnight at room temperature under a dry nitrogen flush. The product, methacryloxyethyl monofluorophosphate, was vacuum distilled to remove any excess difluorophosphoric acid. The residue was filtered through glass wool and collected.

Next, a liner composition was prepared from the following ingredients:

| | Ingredient | Amount, g |
|---|---|---|
| A. | Bis(2-methacryloxyethyl)isophthalate | 2.35 |
| | Bis(2-methacryloxyethyl)phthalate | 1.90 |
| | Bis(2-methacryloxyethyl)terephthalate | 0.75 |
| | Methyl methacrylate | 0.5 |
| | Dodecyl mercaptan | 0.025 |
| | Methacrylic acid | 0.015 |
| | 2,6-di-tertiary butyl *p*-cresol | 0.01 |
| | N,N-dimethyl-3,5-dimethylaniline | 0.035 |
| | Dimethylpolysiloxane | trace |
| | Methacryloxyethyl monofluorophosphate | 0.5 |
| | Gamma-methacrylpropyltrimethoxysilane | 0.025 |
| B. | Acetone | 3.4 |
| | Chloroform | 1.7 |
| | Benzoyl peroxide | 0.06 |

The above liner composition was evaluated using the method of Example 1. The average shear strength of the polymerized composition was 15.6 kg/cm$^2$ on unetched dentin, and 6 kg/cm$^2$ on unetched enamel.

Comparative Example 2

Using the method of Example 1, 5 percent by weight pure difluorophosphoric acid was added to the polymerizable monomer mixture of Example 1, and allowed to stand for 5 days. The resulting adduct was then evaluated as in Example 1. The polymerized resin had an average shear strength of 20 kg/cm$^2$.

If allowed to stand in 37°C distilled water for 42 hours (instead of 24 hours) as in Example 1, the polymerized resin had an average shear strength of 29 kg/cm$^2$.

This Example shows that use of a

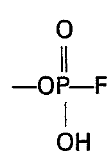

moiety in an adduct with the difunctional methacrylate BIS—GMA gave lower average shear strength values than corresponding BIS—GMA adducts containing moieties such as

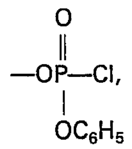 , 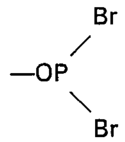 and 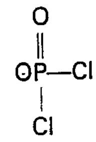 ,

and derived by the addition to BIS—GMA of 5% by weight of phosphorus acids such as $C_6H_5OP(O)Cl_2$, $PBr_3$, or $POCl_3$.

**Claims**

1. A polymerizable composition, characterized by comprising:

(a) at least one polymerizable compound comprising an organic ester of one or more acids of phosphorus, said ester having chlorine or bromine bonded directly to phosphorus, and the organic radical of said ester containing at least one free-radically polymerizable functional group, and

(b) 0.5 to 10 percent by weight of a sulfur compound in the $^{+2}$ or $^{+4}$ oxidation state.

2. A polymerizable composition according to Claim 1, further characterized in that said polymerizable compound has the formula:

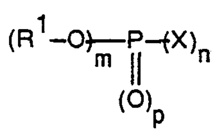 or 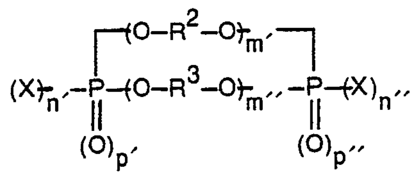

wherein:

m is 1 to 3,

m′ and m″ are zero or 1 and are the same or different with the proviso that m′ and m″ are not both zero,

n is 1 to 4,

n′ and n″ are zero to 4 and are the same or different, with the proviso that n′ and n″ are not both zero,

p, p′, and p″ are zero or 1 and are the same or different,

$m+n+2p=3$ or 5,

$m'+m''+n'+2p'=3$ or 5,

$m'+m''+n''+2p''=3$ or 5,

$R^1$ is a monovalent, olefinic organic radical,

$R^2$ and $R^3$ are divalent, olefinic organic radicals and are the same or different, and

X is Cl, Br, or $R^4$, where $R^4$ is an aliphatic or oxyaliphatic radical having 1 to 12 carbon atoms, and each X is the same as or different from other X, with the proviso that at least one X is Cl or Br.

3. A polymerisable composition according to claim 2 characterised in that $R^1$, $R^2$ and $R^3$ independently contain skeletal hetero atoms selected from oxygen, sulfur and non-basic nitrogen atoms.

4. A polymerisable composition according to any preceding claim, further characterised in that said organic radical is the residue remaining after removal of one or more hydroxyl hydrogen atoms from BIS—GMA.

5. A polymerisable composition according to claim 4, further characterised in that the phosphorus acid is $POCl_3$ and the ratio of said acid to said BIS—GMA is between 0.025:1 and 1:1.

6. A polymerisable composition according to any preceding claim, further characterised in that said polymerisable compound contains

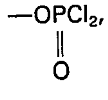 , —$OPCl_2$, 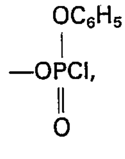 , or —$OPBr_2$ moieties.

7. A polymerisable composition according to any preceding claim, further characterised by containing 0.5 to 10 percent by weight tertiary amine and/or 0.05 to 5 percent by weight free-radical initiator or photoinitiator.

8. A composition for use in dentistry, characterised by comprising:

(a) at least one polymerisable compound comprising the reaction product of a phosphorus acid having

chlorine or bromine bonded directly to phosphorus with a polymerisable monomer having at least one reactive hydroxyl group, and

(b) a sulfur compound in the $^{+2}$ or $^{+4}$ oxidation state.

9. A composition according to claim 8, further characterised by comprising tertiary amine and/or free-radical initiator.

10. A composition according to claim 8, further characterised by comprising a photoinitiator.

11. A composition according to any one of claims 8 to 10, further characterised by the sulfur compound comprising sodium benzenesulfinate.

12. A composition according to any one of claims 8 to 10 further characterised in that said polymerisable compound comprises the reaction product of BIS—GMA in a diluent with 0.25 to 20% by weight phosphorus oxychloride, based on the weight of said BIS—GMA.

13. A composition according to any one of claims 8 to 10 further characterised in that said polymerisable compound comprises the reaction product of hydroxyethyl methacrylate and phosphorus oxychloride.

14. A composition according to claim 13, further characterised in that said hydroxyethyl methacrylate and said phosphorus oxychloride are combined in a ratio of halogen atom to hydroxyl group of between 0.0375:1 to 1.5:1.

15. A two-part polymerisable composition for use as a liner composition, characterised in that the first part comprises the reaction product of BIS—GMA in a diluent with 0.25 to 20 percent by weight phosphorus oxychloride, based on the weight of said BIS—GMA, and a free radical initiator, and the second part comprises a sulfur compound in the $^{+2}$ or $^{+4}$ oxidation state.

16. A two-part composition according to claim 15 wherein the sulphur compound comprises sodium benzenesulfinate.

17. A polymerisable compound, useful in dentistry, characterised by having the formula:

$$(R^1-O)_m-P-(Br)_n$$

or

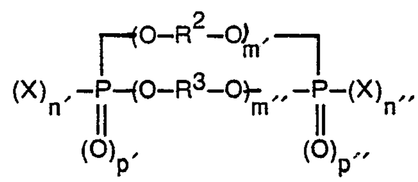

wherein

m and n are at least one,

m+n=3,

m′ and m″ are zero or 1 and are the same or different with the proviso that m′ and m″ are not both zero,

n′ and n″ are zero to 4 and are the same or different with the proviso that n′ and n″ are not both zero,

p′ and p″ are zero or 1 and are the same or different,

$m'+m''+n'+2p'=3$ or 5,

$m'+m''+n''+2p''=3$ or 5,

$R^1$ is a monovalent, olefinic organic radical,

$R^2$ and $R^3$ are divalent, olefinic organic radicals and are the same or different, and

X is Cl, Br, or $R^4$, where $R^4$ is an aliphatic or oxyaliphatic radical having 1 to 12 carbon atoms, and each X is the same as or different from other X, with the proviso that at least one X is Cl or Br.

18. A compound according to claim 17 characterised in that either or both $R^1$ or $R^2$ contain skeletal hetero atoms selected from oxygen, sulfur and non-basic nitrogen atoms.

19. A polymerisable compound useful in dentistry, characterised in that said compound is an organic ester of one or more acids of phosphorus, said ester having chlorine or bromine bonded directly to phosphorus, and the organic radical of said ester containing at least one free-radically polymerisable functional group, wherein said organic radical is the residue remaining after removal of one or more hydroxyl hydrogen atoms from BIS—GMA.

20. A polymerisable compound according to claim 19 further characterised in that phosphorus is doubly bonded to an oxygen atom, is bonded to at least one chlorine atom, and said acid of phosphorus and said BIS—GMA are combined in a ratio of halogen atom to hydroxyl group between 0.0375:1 to 1.5:1.

21. A polymerisable compound useful in dentistry, characterised by comprising an adduct derived by addition to BIS—GMA of a phosphorus acid having chlorine or bromine bonded directly to phosphorus.

22. A compound according to claim 21 further characterised in that said phosphorus acid comprises $POCl_3$, $C_6H_5OP(O)Cl_2$, $PCl_3$, or $PBr_3$.

23. A compound according to claim 21 further characterised in that said adduct contains chlorine and said phosphorus acid comprises phosphorus oxychloride.

24. A method for making a polymerisable compound for use in dentistry, characterised by mixing BIS—GMA with an acid of phosphorus having chlorine or bromine bonded directly to phosphorus.

25. A polymerisable compound as claimed in any one of claims 17 to 23 for use in a method for repair or veneering of hard dental tissue without the necessity for acid etching said tissue.

26. Use of a phosphorus composition for the manufacture of a composition for adhering to dentin characterised in that the phosphorus composition comprises:

(a) at least one polymerisable compound comprising the reaction product of a phosphorus acid having chlorine or bromine bonded directly to phosphorus with a polymerisable monomer having at least one reactive hydroxyl group, and

(b) a sulfur compound in the $^{+2}$ or $^{+4}$ oxidation state.

**Patentansprüche**

1. Polymerisierbare Zusammensetzung, gekennzeichnet durch:

(a) mindestens eine polymerisierbare Verbindung, die mindestens teilweise aus einem organischen Ester einer oder mehrerer Säuren des Phosphors besteht, wobei in dem Ester Chlor oder Brom direkt mit Phosphor verbunden ist und das organische Radikal dieses Esters mindestens eine radikalpolymerisierbare funktionelle Gruppe enthält, und

(b) 0,5 bis 10 Gew.% einer Schwefelverbindung mit der Oxidationszahl $^{+2}$ oder $^{+4}$.

2. Polymerisierbare Zusammensetzung nach Anspruch 1, dadurch gekennzeichnet, daß die polymerisierbare Verbindung die Formel

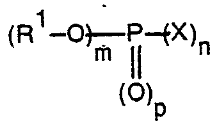 oder 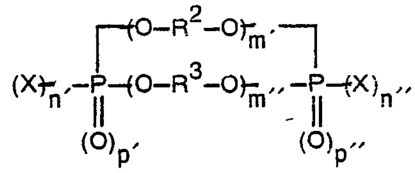

hat, in der

m=1 bis 3 ist,

m′ und m″=null oder 1 und gleich oder verschieden sind, aber m′ und m″ nicht beide gleich null sind,

n=1 bis 4 ist,

n′ und n″=null bis 4 und gleich oder verschieden sind, aber n′ und n″ nicht beide gleich null sind,

p, p′ und p″=null oder 1 und gleich oder verschieden sind,

m+n+2p=3 oder 5 ist,

m′+m″+n′+2p′=3 oder 5 ist,

m′+m″+n″+2p″=3 oder 5 ist,

$R^1$ ein einwertiges olefinisches organisches Radikal ist,

$R^2$ und $R^3$ zweiwertige olefinische organische Radikale und gleich oder verschieden sind und

X=Cl, Br oder $R^4$ ist, wobei $R^4$ ein aliphatisches oder oxyaliphatisches Radikal mit 1 bis 12 Kohlenstoffatomen ist und jedes X jedem anderen X gleich oder von ihm verschieden ist, aber mindestens ein X=Cl oder Br ist.

3. Polymerisierbare Zusammensetzung nach Anspruch 2, dadurch gekennzeichnet, daß $R^1$, $R^2$ und $R^3$ unabhängig voneinander Skelett-Heteroatome enthält, die aus den Sauerstoff- und Schwefelatomen und den nichtbasischen Stickstoffatomen ausgewählt sind.

4. Polymerisierbare Zusammensetzung nach einem der vorhergehenden Ansprüche, dadurch gekennzeichnet, daß das genannte organische Radikal der Rest ist, der nach der Abspaltung eines oder mehrerer Hydroxylwasserstoffatome von Bis-GMA zurückbleibt.

5. Polymerisierbare Zusammensetzung nach Anspruch 4, dadurch gekennzeichnet, daß die Säure des Phosphors wenigstens teilweise aus $POCl_3$ besteht und das Verhältnis dieser Säure zu dem Bis—GMA zwischen 0,025:1 und 1:1 liegt.

6. Polymerisierbare Zusammensetzung nach einem der vorhergehenden Ansprüche, dadurch gekennzeichnet, daß die polymerisierbare Verbindung Anteile der Formeln

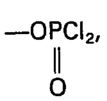, $—OPCl_2$, 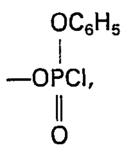, oder $—OPBr_2$ enthält.

7. Polymerisierbare Zusammensetzung nach einem der vorhergehenden Ansprüche, dadurch gekennzeichnet, daß sie 0,5 bis 10 Gew.% tertiäres Amin und/oder 0,05 bis 5 Gew.% eines freie Radikale bildenden Initiators oder Photoinitiators enthält.

8. Zusammensetzung für die Verwendung in der Zahnheilkunde, dadurch gekennzeichnet, daß sie mindestens teilweise aus

(a) mindestens einer polymerisierbaren Verbindung besteht, die ihrerseits mindestens teilweise aus

dem Reaktionsprodukt einer Säure des Phosphors, in der Chlor oder Brom direkt mit Phosphor verbunden ist, mit einem polymerisierbaren Monomer besteht, das mindestens eine reaktionsfähige Hydroxylgruppe enthält, und

(b) aus einer Schwefelverbindung mit der Oxidationszahl $^{+2}$ oder $^{+4}$.

9. Zusammensetzung nach Anspruch 8, dadurch gekennzeichnet, daß sie tertiäres Amin und/oder einen freie Radikale bildenden Initiator enthält.

10. Zusammensetzung nach Anspruch 8, dadurch gekennzeichnet, daß sie einen Photoinitiator enthält.

11. Zusammensetzung nach einem der Ansprüche 8 bis 10, dadurch gekennzeichnet, daß die Schwefelverbindung mindestens teilweise aus Natriumbenzolsulfinat besteht.

12. Zusammensetzung nach einem der Ansprüche 8 bis 10, dadurch gekennzeichnet, daß die polymerisierbare Verbindung mindestens teilweise aus dem Reaktionsproukt von in einem Verdünnungsmittel vorliegenden Bis—GMA mit Phosphoroxychlorid in einer auf das Gewicht des Bis—GMA bezogenen Menge von 0,25 bis 20 Gew.% besteht.

13. Zusammensetzung nach einem der Ansprüche 8 bis 10, dadurch gekennzeichnet, daß die polymerisierbare Verbindung mindestens teilweise aus dem Reaktionsprodukt von Hydroxyethylmethacrylat und Phosphoroxychlorid besteht.

14. Zusammensetzung nach Anspruch 13, dadurch gekennzeichnet, daß das Hydroxyethylmethacrylat und das Phosphoroxychlorid in einem Verhältnis zwischen 0,0375:1 bis 1,5:1 von Halogenatomen zu Hydroxylgruppen miteinander vereinigt sind.

15. Polymerisierbare Zweikomponentenzusammensetzung für die Verwendung als Auskleidungszusammensetzung, dadurch gekennzeichnet, daß die erste Komponente mindestens teilweise aus dem Reaktionsprodukt von in einem Verdünnungsmittel vorliegenden Bis—GMA mit Phosphoroxychlorid in einer auf das Gewicht des Bis—GMA bezogenen Menge von 0,25 bis 20 Gew.% und aus einem freie Radikale bildenden Initiator besteht und die zweite Komponente mindestens teilweise aus einer Schwefelverbindung mit der Oxidationszahl $^{+2}$ oder $^{+4}$ besteht.

16. Zweikomponentenzusammensetzung nach Anspruch 15, dadurch gekennzeichnet, daß die Schwefelverbindung mindestens teilweise aus Natriumbenzolsulfinat besteht.

17. Für die Verwendung in der Zahnheilkunde geeignete polymerisierbare Verbindung, dadurch gekennzeichnet, daß sie die Formel

$(R^1-O)_m-P-(Br)_n$ oder 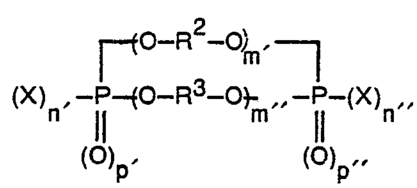

hat, in der

m und n mindestens 1 sind,

m+n=3 ist,

m' und m''=null oder 1 und gleich oder verschieden sind, aber m' und m'' nicht beide gleich null sind,

n' und n''=null bis 4 und gleich oder verschieden sind, aber n' und n'' nicht beide gleich null sind,

p' und p''=null oder 1 und gleich oder verschieden sind,

m+n+2p=3 oder 5 ist,

m'+m''+n'+2p'=3 oder 5 ist,

m'+m''+n''+2p''=3 oder 5 ist,

$R^1$ ein einwertiges olefinisches organisches Radikal ist,

$R^2$ und $R^3$ zweiwertige olefinische organische Radikale und gleich oder verschieden sind und

X=Cl, Br oder $R^4$ ist, wobei $R^4$ ein aliphatisches oder oxyaliphatisches Radikal mit 1 bis 12 Kohlenstoffatomen ist und jedes X jedem anderen X gleich oder von ihm verschieden ist, aber mindestens ein X=Cl oder Br ist.

18. Verbindung nach Anspruch 17, dadurch gekennzeichnet, daß $R^1$ und/oder $R^2$ Skelett-Heteroatome enthalten, die aus den Sauerstoff- und Schwefelatomen und den nichtbasischen Stickstoffatomen ausgewählt sind.

19. Für die Verwendung in der Zahnheilkunde geeignete polymerisierbare Verbindung, dadurch gekennzeichnet, daß die Verbindung ein organischer Ester einer oder mehrerer Säuren des Phosphors ist, wobei in dem Ester Chlor oder Brom direkt mit Phosphor verbunden ist und das organische Radikal dieses Esters mindestens eine radikalpolymerisierbare funktionelle Gruppe enthält und das genannte organische Radikal der Rest ist, der nach der Abspaltung eines oder mehrerer Hydroxylwasserstoffatome von Bis—GMA zurückbleibt.

20. Polymerisierbare Verbindung nach Anspruch 19, dadurch gekennzeichnet, daß Phosphor durch eine Doppelbindung mit einem Sauerstoffatom verbunden ist und mit mindestens einem Chloratom verbunden ist und daß die Säure des Phosphors und das Bis—GMA in einem Verhältnis von Halogenatomen zu Hydroxylgruppen zwischen 0,0375:1 und 15:1 miteinander vereinigt sind.

21. Für die Verwendung in der Zahnheilkunde geeignete polymerisierbare Verbindung, dadurch

gekennzeichnet, daß sie mindestens teilweise aus einem Addukt besteht, daß durch den Zusatz einer Säure des Phosphors, in der Chlor oder Brom direkt mit Phosphor verbunden sind, zu Bis—GMA gewonnen worden ist.

22. Verbindung nach Anspruch 21, dadurch gekennzeichnet, daß die Saüre des Phosphors aus $POCl_3$, $C_6H_5OP(O)Cl_2$, $PCl_3$ oder $PBr_3$ enthält.

23. Verbindung nach Anspruch 21, dadurch gekennzeichnet, daß das Addukt Chlor enthält und die Säure des Phosphors Phosphoroxychlorid enthält.

24. Verfahren zum Erzeugen einer für die Verwendung in der Zahnheilkunde geeigneten polymerisierbaren Verbindung, dadurch gekennzeichnet, daß Bis—GMA mit einer Säure des Phosphors gemischt wird, in der Chlor oder Brom direkt mit Phosphor verbunden sind.

25. Polymerisierbare Verbindung nach einem der Ansprüche 17 bis 23 für die Verwendung in einem Verfahren zum Instandsetzen oder Überziehen von hartem Zahngewebe, ohne daß dieses mit Säure geätzt werden muß.

26. Verwendung einer phosphorhaltigen Zusammensetzung für die Herstellung einer Zusammensetzung, die an Dentin haftet, dadurch gekennzeichnet, daß die phosphorhaltige Zusammensetzung mindestens teilweise besteht aus

(a) mindestens einer polymerisierbaren Verbindung, die mindestens teilweise aus dem Reaktionsprodukt einer Säure des Phosphors, in der Chlor oder Brom direkt mit Phosphor verbunden sind, mit einem polymerisierbaren Monomer besteht, das mindestens eine reaktionsfähige Hydroxylgruppe besitzt, und aus

(b) einer Schwefelverbindung mit der Oxidationszahl $^{+2}$ oder $^{+4}$.

**Revendications**

1. Composition polymérisable caractérisée en ce qu'elle comprend:

(a) au moins un composé polymérisable comprenant un ester organique d'un ou plusieurs acides phosphorés, ledit ester ayant du chlore ou du brome directement lié au phosphore, et le radical organique dudit ester ayant au moins un groupe fonctionnel radicalairement polymérisable, et

(b) 0,5 à 10% en poids d'un composé soufré où le soufre est à l'état d'oxydation de $^{+2}$ ou $^{+4}$.

2. Composition polymérisable selon la revendication 1, caractérisée en outre par le fait que ledit composé polymérisable répond aux formules

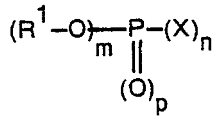

ou

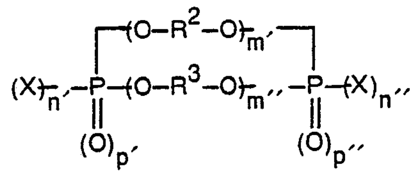

dans lesquelles

m est 1 à 3,

m' et m'' sont 0 ou 1 et sont identiques ou différents avec la condition que m' et m'' ne soient pas tous deux zéro,

n est 1 à 4,

n' et n'' sont 0 à 4 et sont identiques ou différents, sous réserve que n' et n'' ne soient pas tous deux zéro,

p, p' et p'' sont 0 ou 1 et sont identiques ou différents,

$m+n+2p=3$ ou 5,

$m'+m''+n'+2p'=3$ ou 5,

$m'+m''+n''+2p''=3$ ou 5,

$R^1$ est un radical organique oléfinique monovalent,

$R^2$ est $R^3$ sont des radicaux organiques oléfiniques divalents et sont identiques ou différents,

X est Cl, Br ou $R^4$, où $R^4$ est un radical aliphatique ou oxyaliphatique ayant de 1 à 12 atomes de carbone, et chaque X est identique ou différent d'un autre X, sous réserve qu'au moins un des X soit Cl ou Br.

3. Composition polymérisable selon la revendication 2, caractérisée en ce que $R^1$, $R^2$ et $R^3$ indépendamment contiennent dans leur squelette des hétéro-atomes choisis parmi les atomes d'oxygène, de soufre et d'azote non basique.

4. Composition selon l'une quelconque des revendications précédentes, caractérisée en ce que ledit radical organique est le reste demeurant après élimination d'un ou plusieurs atoms d'hydrogène hydroxylique du BIS—GMA.

5. Composition selon la revendication 4, caractérisée en ce que l'acide phosphorique est $POCl_3$ et le rapport dudit acide audit BIS—GMA est situé entre 0,025/1 et 1/1.

6. Composition polymérisable selon l'une quelconque des revendications précédentes, caractérisée en ce que ledit composé polymérisable contient des fragments

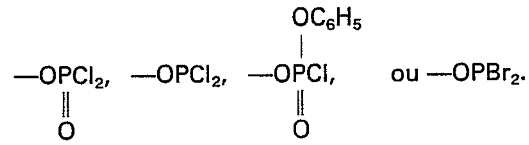

, , ou

7. Composition polymérisable selon l'une quelconque des revendications précédentes, caractérisée en outre par le fait qu'elle contient 0,5 à 10% en poids d'amine tertiaire et/ou 0,05 à 5% en poids d'amorceur radicalaire ou photoinitiateur.

8. Composition utile dans le domaine de la dentisterie, caractérisée en ce que qu'elle comprend

(a) au moins un composé polymérisable comprenant le produit de réaction d'un acide phosphoré ayant du chlore ou du brome directement lié au phosphore avec un monomère polymérisable ayant au moins un groupe OH réactif, et

(b) un composé soufré à l'état d'oxydation $^{+2}$ ou $^{+4}$.

9. Composition selon la revendication 8, caractérisée en outre par le fait qu'elle comprend une amine tertiare et/ou un amorceur radicalaire.

10. Composition selon la revendication 8, caractérisée en outre par le fait qu'elle comprend un photoinitiateur.

11. Composition selon l'une quelconque des revendications 8 à 10, caractérisée en outre par le fait que le composé soufré comprend le benzènesulfinate de sodium.

12. Composition selon l'une quelconque des revendications 8 à 10, caractérisée en outre par le fait que ledit composé polymérisable comprend le produit de réaction du BIS—GMA dans un diluant avec 0,25 à 20% en poids de $POCl_3$ par rapport au poids dudit BIS—GMA.

13. Composition selon l'une quelconque des revendications 8 à 10, caractérisée en outre par le fait que ledit composé polymérisable comprend le produit de réaction du méthacrylate d'hydroxyéthyle et du $POCl_3$.

14. Composition selon la revendication 13, caractérisée en outre par le fait que ledit méthacrylate d'hydroxyéthyle et ledit $POCl_3$ sont combinés suivant un rapport atome d'halogène/groupe OH compris entre 0,0375/1 et 1,5/1.

15. Composition polymérisable à deux composants pour utilisation en tant que composition de placage, caractérisée en ce que le premier composant comprend le produit de réaction du BIS—GMA dans un diluant avec 0,25 à 20% en poids de $POCl_3$ par rapport au poids dudit BIS-GMA, et un amorceur radicalaire, et le second composant comprend un composé soufré à l'état d'oxydation $^{+2}$ ou $^{+4}$.

16. Composition à deux composants selon la revendication 15, dans laquelle le composé soufré comprend le benzènesulfinate de sodium.

17. Composé polymérisable utile en dentisterie, caractérisé en ce qu'il répond aux formules

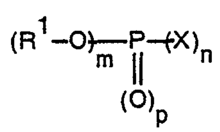

ou

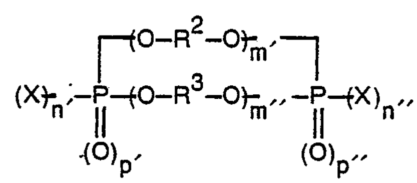

dans lesquelles

m est 1 à 3,

m' et m'' sont 0 ou 1 et sont identiques ou différents avec la condition que m' et m'' ne soient pas tous deux zéro,

n est 1 à 4,

n' et n'' sont 0 à 4 et sont identiques ou différents, sous réserve que n' et n'' ne soient pas tous deux zéro,

p, p' et p'' sont 0 ou 1 et sont identiques ou différents,

$m+n+2p=3$ ou 5,

$m'+m''+n'+2p'=3$ ou 5,

$m'+m''+n''+2p''=3$ ou 5,

$R^1$ est un radical organique oléfinique monovalent,

$R^2$ est $R^3$ sont des radicaux organiques oléfiniques divalents et sont identiques ou différents,

X est Cl, Br ou $R^4$, où $R^4$ est un radical aliphatique ou oxyaliphatique ayant de 1 à 12 atomes de carbone, et chaque X est identique ou différent d'un autre X, sous réserve qu'au moins un des X soit Cl ou Br.

18. Composé selon la revendication 17, caractérisé en ce que l'un des $R^1$ ou $R^2$ ou chacun des $R^1$ et $R^2$ contient dans son squelette des hétéroatomes choisis parmi les atomes d'oxygène, de soufre et d'azote non basique.

19. Composé polymérisable utile en dentisterie, caractérisé en ce que ledit composé est un ester organique d'un ou plusieurs acides phosphorés, ledit ester ayant du chlore ou du brome directement lié au phosphore, et le radical organique dudit ester ayant au moins un groupe fonctionnel radicalairement polymérisable, dans lequel ledit radical organique est le reste demeurant après élimination d'un ou plusieurs atomes d'hydrogène hydroxylique du BIS—GMA.

20. Composé polymérisable selon la revendication 19, caractérisé en outre par le fait que le phosphore est lié à un atome d'oxygène par une double liaison, est lié à au moins un atome de chlore, et par le fait que ledit acide phosphoré et ledit BIS—GMA sont combinés suivant un rapport atome d'halogène/groupe OH compris entre 0,0375/1 et 1,5/1.

21. Compose polymérisable utile en dentisterie, caractérisé en ce qu'il comprend un adduct provenant de l'addition au BIS—GMA d'un acide phosphoré ayant du chlore ou du brome directement lié au phosphore.

22. Composé selon la revendication 21, caractérisé en outre par le fait que ledit acide phosphoré comprend $POCl_3$, $C_6H_5OP(O)Cl_2$, $PCl_3$ ou $PBr_3$.

23. Composé selon la revendication 21, caractérisé en outre par le fait que ledit adduct contient du chlore et ledit acide phosphoré comprend $POCl_3$.

24. Procédé de préparation d'un composé polymerisable utile en dentisterie, caractérisé par le mélange du BIS—GMA avec un acide phosphoré ayant du chlore ou du brome directement lié au phosphore.

25. Composé polymérisable selon l'une quelconque des revendications 17 à 23, pour utilisation dans un procédé de réparation ou restauration du tissu dentaire dur sans besoin d'une attaque acide dudit tissu.

26. Utilisation d'une composition phosphorée dans l'obtention d'une composition adhérant à la dentine, caractérisée en ce que la composition phosphorée comprend:

(a) au moins un composé polymérisable comprenant le produit de réaction d'un acide phosphoré ayant du chlore ou du brome directement lié au phosphore avec un monomère polymérisable ayant au moins un groupe OH réactif, et

(b) un composé soufré à l'état d'oxydation $^{+2}$ ou $^{+4}$.